# EUROPEAN PATENT APPLICATION

(11) **EP 3 937 177 A1**
(43) Date of publication of application: **12.01.2022**
(21) Application number: 19918443.3
(22) Date of filing: 27.03.2019
(51) Int. Cl.: G16B 30/10, G16B 30/20, G16B 15/00

(54) **DNA CODING METHOD AND BIOMEDICAL ENGINEERING APPLICATION OF SAME CODING METHOD**

(30) Priority: 05.03.2019 KR 20190025377
(71) Applicant: Hessegg, Inc., Seoul 05855 (KR)
(72) Inventor: KIM, Hyun Ju, Seoul 08793 (KR); SON, In Sik, Seongnam-Si Gyeonggi-do 13614 (KR)
(74) Representative: FRKelly
(86) International application number: PCT/KR2019/003570
(87) International publication number: WO 2020/179962

(57) **Abstract**

The present invention relates to a method for code standardizing DNA (a) C, T, A, and G are designated as 00, 01, 10, and 11, respectively, and (b) when each base is a base pair of G and C and A and T, in the 5' to 3' direction, designated as 1100 for G and C, 0011 for C and G, and 1001 for A and T and 0110 for T and A. As a result, the DNA code standardization method of the present invention provides an easy method for identifying specific patterns, secondary structures, and nucleotide sequence variations within the nucleotide sequence, and facilitates the prediction of diseases by using disease-specific sequence mutations such as SNPs. It provides an easy method for identifying a specific pattern present in a nucleotide sequence such as a DNA fragment or an aptamer.

## Description

### [Technical Field]

The present invention relates to a DNA coding method and biomedical application thereof.

### [Background Art]

DNA (Deoxyribonucleic Acid), which exists as genetic material in living things, is composed of a gene portion expressed as a protein and a non-gene portion. The chemical structure of DNA is that a phosphate group is linked to the 5' carbon of the pentose, which is deoxyribose, and a base is linked to the 1' carbon to form a unit called nucleotide. The DNA sequence is determined according to the type of base linked to the nucleotide.

There are two types of bases, the purine base having two ring structures and the pyrimidine series having one ring structure. The purine series includes guanine (G) and adenine (A), and the pyrimidine series includes cytosine (C) and thymine (T). There is a difference in RNA that it is substituted with uracil (U) instead of thymine. G of the purine series forms a complementary pair with C, a pyrimidine, by hydrogen bonding, and A pairs with T. Since the complementary bond between G and C is connected by three hydrogen bonds, it forms a stronger bond than the bond between A and T that forms two hydrogen bonds.

In the nucleotide unit of DNA, a phosphate group connected to the 5' carbon is connected to the 3' carbon -OH group of another unit by a phosphate diester bond to form a single strand. Two complementary single strands linked by a phosphate diester bond form a double helix structure by hydrogen bonding of complementary bases. This double helix structure was introduced by Watson and Crick in 1953. [Watson, J. D., & Crick, F. H. (1953). Molecular structure of nucleic acids. Nature, 171(4356), 737-738.]

The nucleotide sequence of the gene region in DNA plays an important role in protein synthesis as the three nucleotide codes are translated into one amino acid constituting the protein. After DNA is transcribed into mRNA, it is translated into 20 amino acids according to the sequence of the base sequence, and the translated amino acids are linked by tRNA to form a protein. The protein exists as a component in cells and also acts as an enzyme mediating various reactions in the body.

Human DNA has 3 billion base pairs (bp) and has a data capacity of GB per person. When this capacity is converted into the number of people, even PB units are insufficient. Therefore, rather than analyzing all human DNA sequences, disease prediction analysis is performed with a sequence of short DNA fragments by analyzing disease-specific SNP (Single Nucleotide Polymorphism) sites, etc., but even this does not analyze the SNP sites of all genes. and it is necessary to develop various programs to analyze it.

### [Prior patent literature]

Korean Patent Publication No. 10-2016-0001455

### [Disclosure]

### [Technical Problem]

The present invention solves the above problems and is due to the necessity of the above, an object of the present invention provide a method optimized for identifying a specific pattern existing in a nucleotide sequence by standardizing a DNA base into a binary code (2 bits per base) in consideration of the molecular weight of each base.

Another object of the present invention is to provide an easy method for detecting whether complementary binding and a pattern using a code sum of a nucleotide sequence and a method for predicting the pattern and function of a DNA fragment or a DNA aptamer.

Another object of the present invention is to provide an easy method for determining the molecular weight ratio between sequences and the ratio of each base only with the code of the base sequence.

Another object of the present invention is to provide an easy method for detecting a mutation in a nucleotide sequence and to provide an easy method for predicting a disease by using a disease-specific sequence mutation such as SNP

### [Technical Solution]

In order to achieve the above object, the present invention provides a method of standardizing DNA to code comprising the steps of:
(a) C, T, A, and G are designated as 00, 01, 10, and 11, respectively; and
(b) When each base is paired with G and C and A and T, in the 5' to 3' direction, it is designated as 1100 for G and C, 0011 for C and G, and 1001 for A and T and 0110 for T and A.

And the present invention provides a method of providing information optimized to identify a specific pattern or secondary structure of a specific DNA fragment or aptamer using DNA code standardization, comprising the steps of:
(a) designating C, T, A, and G of a specific DNA fragment nucleotide sequence as 00, 01, 10, 11, respectively; and
(b) comparing the numerically designated code arrangement with the code sum arrangement.

In one embodiment of the present invention, the step of comparing the numerically designated code arrangement with the code sum arrangement is preferable in that it is determined that a stem structure is formed when two or more pairs of codes whose sum of each sequence becomes 3 after transforming the binary number sequence of 00, 01, 10, and 11 in step (a) into a decimal number are arranged at both ends, and a loop structure is formed when three or more sequences that cannot form complementary binding are linked to the center because the code sum of the sequences facing each other is greater than or smaller than 3, but not limited thereto.

The present invention provides a method of providing information on the presence or absence of nucleotide sequence variation in a specific DNA fragment using DNA code standardization comprising the steps of:
(a) designating C, T, A, and G of a specific DNA fragment nucleotide sequence as 00, 01, 10, 11, respectively; and
(b) comparing the sum of the numerically designated codes.

In one embodiment of the present invention, the step of comparing of the sum of the codes is preferable in that it is determined that mutation is present when there is a difference of 1 to 3 after transforming the binary number arrangement of 00, 01, 10, and 11 in step (a) into a decimal number, obtaining the sum and then comparing it with a normal sequence, but not limited thereto.

In another embodiment of the present invention, the position of the variant sequence is confirmed by preferably comparing each value of the codes obtained by designating C, T, A, and G of the nucleotide sequence of a specific DNA fragment as 00, 01, 10, and 11, respectively, but not limited thereto.

The present invention provides an information providing computer program, stored in a computer-readable medium, optimized for identifying a specific pattern or secondary structure of a specific DNA fragment or aptamer for causing a computer to perform the following steps, the steps of:
(a) designating C, T, A, and G of the nucleotide sequence of a specific DNA fragment as 00, 01, 10, 11, respectively; and
(b) it is determined that a stem structure is formed when two or more pairs of codes whose sum of each sequence becomes 3 after transforming the binary number sequence of 00, 01, 10, and 11 in step (a) into a decimal number are arranged at both ends, and a loop structure is formed when three or more sequences that cannot form complementary binding are linked to the center because the code sum of the sequences facing each other is greater than or smaller than 3.

The present invention provides a computer program, stored in a computer-readable medium, for providing information on whether a nucleotide sequence variation exists in a specific DNA fragment for causing a computer to perform the following steps, the steps of:
(a) designating C, T, A, and G of the nucleotide sequence of a specific DNA fragment as 00, 01, 10, 11, respectively; and
(b) it is determined that mutation is present when there is a difference of 1 to 3 after transforming the binary number arrangement of 00, 01, 10, and 11 in step (a) into a decimal number, obtaining the sum and then comparing it with a normal sequence.

The present invention provides a computer program stored in a computer-readable medium for providing information on the position of a nucleotide sequence variation sequence of a specific DNA fragment for causing a computer to perform the following steps, the steps of:
(a) designating C, T, A, and G of the nucleotide sequence of a specific DNA fragment as 00, 01, 10, 11, respectively; and
(b) it is determined that the position of the variant sequence is confirmed by comparing each value of the codes obtained by designating C, T, A, and G of the nucleotide sequence of a specific DNA fragment as 00, 01, 10, and 11, respectively.

Hereinafter, the present invention will be described.

In the present invention, it provides a method of designating codes comprising each of the DNA's four bases, C, T, A, and G, in descending order of molecular weight, are designated as codes 00, 01, 10, and 11, respectively so that when each base is paired with G and C and A and T, the sum of the molecular weights corresponds to the ratio of the code sum.

In addition, the present invention establishes a system that can be used as big data to identify a specific pattern of binding to a reactive group present in each compound and predict it by standardizing the aptamer specific to each compound identified using SELEX as a code.

In addition, the present invention provides a method for checking the presence or absence of mutations in each sequence and quickly determining the presence or absence of a SNP of a specific disease by standardizing the DNA sequence as a code, converting the value of each sequence to a decimal number, and deriving the sum thereof.

The present invention provides an easy method for identifying a specific pattern present in a nucleotide sequence by standardizing DNA into a code.

The present invention provides the necessary information for SELEX (Systematic evolution of ligands by exponential enrichment) simulation program and for predicting an aptamer that binds to a corresponding chemical structural unit by identifying a DNA sequence pattern binding to a specific target and chemical structure and utilizing it as big data.

In addition, the present invention provides a method optimized for determining the molecular weight ratio between sequences and the ratio of each base only with the code of the base sequence by standardizing DNA with a code reflecting the molecular weight of the base.

In addition, the present invention provides an easy method for identifying mutations in the nucleotide sequence by standardizing DNA with a code that reflects the molecular weight of the base and provides an optimized method for comparing the sum of codes and sequence arrangement order, thereby identifying disease-specific mutations such as SNPs and provides an easy method for disease prediction.

### [Advantageous Effects]

As can be seen through the present invention, the DNA code standardization method of the present invention provides an easy method to identify mutations in the nucleotide sequence and provides an easy method for identifying a specific pattern present in a nucleotide sequence, such as facilitating the prediction of a disease by using a disease-specific sequence variation such as SNP.

### [Description of Drawings]

Figure 1 shows that the code values designated by reflecting the principle of the molecular structure and binding mass ratio of DNA are designated as binary numbers of 00, 01, 10, and 11 values of C, T, A, and G in the order from the smallest to the largest in molecular weight;
Figure 2 is a diagram showing that the designated binary code is designed so that when the bases of G and C, A and T are paired, the ratio of the sum of each code is 1:1, and is designed to have the same ratio as the actual mass ratio;
Figure 3 is a diagram showing the code conversion values of six sequences, comparing the code sum of each sequence and the molecular weight of each sequence;
Figure 4 confirms the pattern of the exemplary sequence using the code of the DNA sequence, confirming whether complementary binding is possible according to the code sum of each sequence, and confirming the stem-loop structure formation and pattern according to the number of bonds and the number of connected bases, and
Figure 5 shows the code standardization efficiency of the present invention by applying the code to the SNP sequence identified in breast cancer patients. The SNP sequence in which the A base at the 14th position from Exon 2 is mutated to G is converted into a code and after placing them as a binary number arrangement, comparing the code sum of the normal sequence and the mutant sequence by obtaining the code sum.

### [Mode for Invention]

Hereinafter, the present invention will be described in more detail by the following examples. However, the following examples are described with the intention of illustrating the present invention, and the scope of the present invention is not to be construed as being limited by the following examples.

### Example 1: Code standardization according to the molecular weight of each base

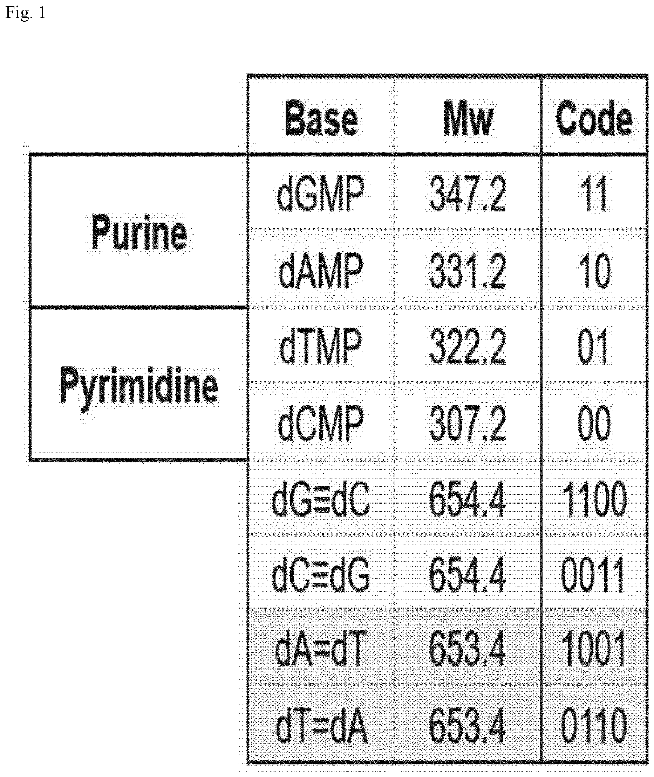

Each of the four bases determining the sequence of DNA was expressed as a binary two-digit number, which is a computer language, and the molecular weight of each base was analyzed and indicated in FIG. 1 to standardize the code. Each of the bases G, A, T, C and a deoxyribonucleotide linked to one phosphate group were denoted as dGMP, dAMP, dTMP, and dCMP, respectively.

Each base has the largest value in the order of G, A, T, and C. if comparing by adding the molecular weights of C, which is paired with G by a hydrogen bond, and T, which is complementary to A, 654.4 (=347.2+307.2) and 653.4 (=331.2+322.2), confirming that they are paired with each other with an equivalent molecular mass of approximately 1:1. The reason that the sum of the molecular weights of A and T is 1 less than the sum of the molecular weights of G and C is that G=C has nitrogen (N), A=T has carbon (C), and hydrogen (H) by one compared to other bonding pairs. It is because there is a difference (=1) of the sum of the molecular weights of each pair as much as the difference between the molecular weight of N and the sum of the molecular weights of C + H (14>12+1). Therefore, A and T form two hydrogen bonds in the absence of O or N capable of hydrogen bonding, thereby forming a weaker bond than the G=C bond, which forms three hydrogen bonds. Therefore, the code of each base was designated by reflecting the principle of the molecular structure and binding mass ratio of the DNA. The codes of each given base were designated as binary numbers of 00, 01, 10, and 11 values for C, T, A, and G in the order from the smallest to the largest in molecular weight. (Fig. 1)

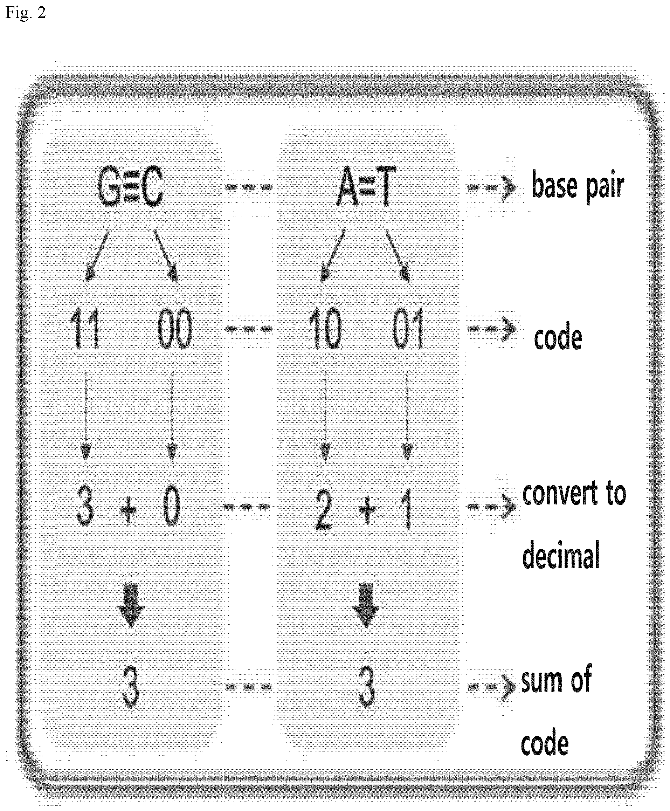

The designated code value is designed so that when the bases of G and C and A and T are paired, the code sum ratio is 1:1, which is the same as the actual mass ratio. (Fig. 2)

The code sum is the sum of each code value after converting the code of each base into a decimal number. The code sum of G and C and A and T is the same as '3'.

### Example 2: Optimization of reflection of molecular weight ratios of DNA fragments and aptamers

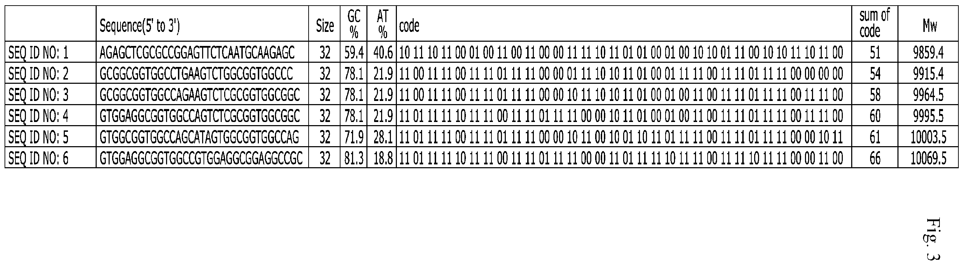

Since according to the molecular weight of each base of DNA the codes were assigned in the order of mass from lowest to highest, the total code sum of the DNA fragments was calculated by reflecting the ratio of the molecular weights of each sequence. (FIG. 3) By checking the molecular weight reflection ratio of the code, the code sum and molecular weight were compared with 6 exemplary sequences.

The exemplary sequence is a sequence exemplified for the purpose of confirming the molecular weight reflection ratio of the code, and the scope of the present invention is not to be construed as being limited to the sequence of SEQ ID NOs: 1 to 6.

The sequences of SEQ ID NOs: 1 to 6 are as follows.
5' AGAGCTCGCGCCGGAGTTCTCAATGCAAGAGC 3' (SEQ ID NO: 1)
5' GCGGCGGTGGCCTGAAGTCTGGCGGTGGGCCCC 3' (SEQ ID NO: 2)
5' GCGGCGGTGGCCAGAAGTCTCGCGGTGGCGGC 3' (SEQ ID NO: 3)
5' GTGGAGGCGGTGGCCAGTCTCGCGGTGGCGGC 3' (SEQ ID NO: 4)
5' GTGGCGGTGGCCAGCATAGTGGCGGTGGGCCAG 3' (SEQ ID NO: 5)
5' GTGGAGGCGGTGGCCGTGGAGGCGGAGGCCGC 3' (SEQ ID NO: 6)

The six exemplary sequences are 32 mer nucleotide sequences, the length of the nucleotides is the same, but the types and sequences of the nucleotides are variously configured, and the code conversion values of each nucleotide are indicated in FIG. 3 . The code sum was calculated by converting the code of each base into a decimal number and according to the base characteristic of each sequence, the code sum was also calculated by reflecting the molecular weight of each sequence.

When compared with the molecular weight (M.W.) of each sequence, the smaller the molecular weight, the smaller the code sum was, and the higher the molecular weight, the higher the code sum was. (Fig. 3)

In this way, codes were designated by reflecting the ratio of molecular weights and was optimized to compare the ratios of molecular weights of each sequence by using the resultant conversed code sum.

### Example 3: Optimization of pattern identification of DNA fragments and aptamers

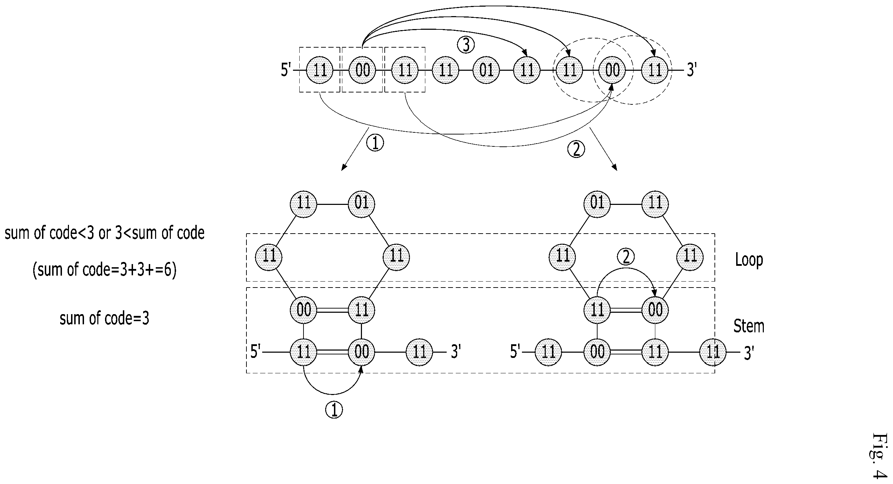

By converting the sequences of DNA fragments and aptamers into binary nucleotide codes and comparing each sequence, it was optimized to identify specific patterns and secondary structures included in the sequences. To understand this, a DNA sequence consisting of 9 nucleotide sequences was used as an example sequence. (Fig. 4)

The above exemplary sequence is intended to illustrate the pattern of the code, and the scope is not to be construed as being limited to the exemplary sequence of SEQ ID NO: 7.

An exemplary sequence of SEQ ID NO: 7 is as follows.
5' GCGGTGGCG 3' (SEQ ID NO: 7)

The number listed by converting the example sequence into a nucleotide code is as follows.
11 00 11 11 01 11 11 00 11 (Example sequence code 1)

The code is designed so that each base has a code sum of '3' with a complementary base capable of forming hydrogen bonding, and the arrangement of these sequences can form a stem structure in the DNA aptamer sequence. (Fig. 4; Stem)

Most of the stem-loop structure patterns of DNA have two or more bases that can form a stem structure at both ends. Since the code sum of the sequences facing each other is greater than or less than 3, there is a characteristic that a loop structure can be formed when three or more sequences that cannot form complementary binding are linked in the center.

The exemplary sequence may form two stem-loop structures, which can be simply confirmed by a base code arrangement. The sequence capable of complementary binding with the first 11 base code is the base of the 8th 00 code except for the 00 code next to it (Fig. 4; ① red arrow) and bases capable of complementary binding to the second 00 code include the 6th 11 (Fig. 4; ③ green arrow) and the 7th 11 and 9th 11 codes. Similarly, the base of the 3rd 11 code can be complementary to the 8th 00 (Fig. 4; ②blue arrow) code. At this time, since the stem region of the stem-loop structure forms a structure only when two or more bases are connected, the complementary bond of the base connected to the red arrow or the complementary bond of the base connected to the blue arrow in Fig. 3 may form the stem structure (Fig. 4) ; dotted round circle), and the complementary bond indicated by the green arrow cannot form a stem structure as a single complementary bond. In both cases that can form a stem structure, since four bases capable of forming a loop structure exist in the middle, it is predicted that the stem-loop structure can be formed.

In this way, by standardizing each base as a code, it is possible to predict whether or not complementary binding to each base is possible according to the base code sum, and it was confirmed that it was easy to predict the secondary structure and pattern of the DNA sequence according to the number of complementary bonds of each sequence and the number of bases connected thereto.

### Example 4: Optimization of SNP identification due to code standardization

By converting the DNA sequence into a code and comparing the code sum of each sequence, it was optimized to determine whether the nucleotide sequence of a specific DNA fragment is mutated. Since the SNP sequence is a DNA fragment sequence in which one base has been mutated, it was confirmed that the code was applied to the SNP sequence and compared with the normal sequence, thereby making it easy to determine the presence and location of the mutation. The efficiency of code standardization was confirmed by applying it to the SNP sequence of the CD44 gene, which is one of various SNP sequences and is found in 84% of breast cancer patients. [Zhou, J., Nagarkatti, P. S., Zhong, Y., Creek, K., Zhang, J., & Nagarkatti, M. (2010). Unique SNP in CD44 intron 1 and its role in breast cancer development. Anticancer research, 30(4), 1263-1272.]

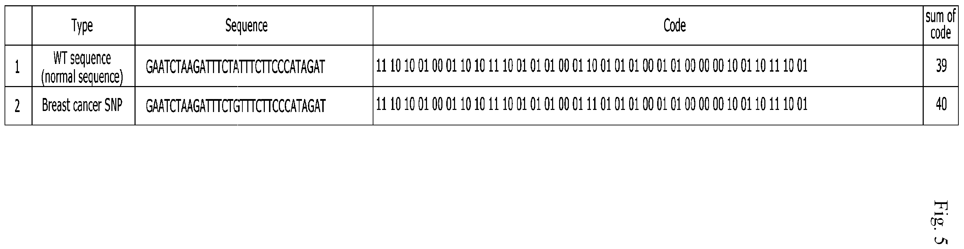

The SNP sequence of the breast cancer patient is a sequence in which the A base at the 14th position from the exon (Exon 2) is mutated to G among the sequences present at the position of the first intron (intron 1) of the gene. This sequence was converted into a code, arranged in a binary array, and the code sum was calculated, and the code sum of the normal sequence and the mutant sequence was compared. (Fig. 5)

When the codes of the normal sequence and the mutant sequence were transformed into decimal numbers, respectively, and the sum was calculated, the normal sequence was 39, the mutant sequence was 40, and the mutant sequence was confirmed as a value 1 greater than the normal sequence. In this way, it is possible to determine whether a mutation exists in a DNA fragment only with the code sum, and at this time, the code sum may differ by 1 to 3 depending on the type of mutated base. In addition, the position of the sequence can be confirmed by comparing the values of each of the mutated codes.

As described above, by converting the DNA fragment sequences identified in the normal control group and the specific mutant sequence identified in the disease test group into codes and comparing the code sum, the difference between the sequences can be quickly identified and the existence of SNPs can be easily searched for, and by applying a code sum to the identified SNP sequence, it can be used for disease diagnosis.

## Claims

1. A method of standardizing DNA to code comprising the steps of:
(a) C, T, A, and G are designated as 00, 01, 10, and 11, respectively; and
(b) when each base is paired with G and C and A and T, in the 5' to 3' direction, it is designated as 1100 for G and C, 0011 for C and G, and 1001 for A and T and 0110 for T and A.

2. A method of providing information optimized to identify a specific pattern or secondary structure of a specific DNA fragment or aptamer using DNA code standardization, comprising the steps of:
(a) designating C, T, A, and G of a specific DNA fragment nucleotide sequence as 00, 01, 10, 11, respectively; and
(b) comparing the numerically designated code arrangement with the code sum arrangement.

3. The method according to claim 2, wherein the step of comparing the numerically designated code arrangement with the code sum arrangement is **characterized in that** it is determined that a stem structure is formed when two or more pairs of codes whose sum of each sequence becomes 3 after transforming the binary number sequence of 00, 01, 10, and 11 in step (a) into a decimal number are arranged at both ends, and a loop structure is formed when three or more sequences that cannot form complementary binding are linked to the center because the code sum of the sequences facing each other is greater than or smaller than 3.

4. A method of providing information on the presence or absence of nucleotide sequence variation in a specific DNA fragment using DNA code standardization comprising the steps of:
(a) designating C, T, A, and G of a specific DNA fragment nucleotide sequence as 00, 01, 10, 11, respectively; and
(b) comparing the sum of the numerically designated codes.

5. The method of claim 4, wherein the step of comparing of the sum of the codes is **characterized in that** it is determined that mutation is present when there is a difference of 1 to 3 after transforming the binary number arrangement of 00, 01, 10, and 11 in step (a) into a decimal number, obtaining the sum and then comparing it with a normal sequence.

6. The method according to claim 4, wherein the position of the variant sequence is confirmed by comparing each value of the codes obtained by designating C, T, A, and G of the nucleotide sequence of a specific DNA fragment as 00, 01, 10, and 11, respectively.

7. An information providing computer program, stored in a computer-readable medium, optimized for identifying a specific pattern or secondary structure of a specific DNA fragment or aptamer for causing a computer to perform the following steps, the steps of:
(a) designating C, T, A, and G of the nucleotide sequence of a specific DNA fragment as 00, 01, 10, 11, respectively; and
(b) it is determined that a stem structure is formed when two or more pairs of codes whose sum of each sequence becomes 3 after transforming the binary number sequence of 00, 01, 10, and 11 in step (a) into a decimal number are arranged at both ends, and a loop structure is formed when three or more sequences that cannot form complementary binding are linked to the center because the code sum of the sequences facing each other is greater than or smaller than 3.

8. A computer program, stored in a computer-readable medium, for providing information on whether a nucleotide sequence variation exists in a specific DNA fragment for causing a computer to perform the following steps, the steps of:
(a) designating C, T, A, and G of the nucleotide sequence of a specific DNA fragment as 00, 01, 10, 11, respectively; and
(b) it is determined that mutation is present when there is a difference of 1 to 3 after transforming the binary number arrangement of 00, 01, 10, and 11 in step (a) into a decimal number, obtaining the sum and then comparing it with a normal sequence.

9. A computer program stored in a computer-readable medium for providing information on the position of a nucleotide sequence variation sequence of a specific DNA fragment for causing a computer to perform the following steps, the steps of:
(a) designating C, T, A, and G of the nucleotide sequence of a specific DNA fragment as 00, 01, 10, 11, respectively; and
(b) it is determined that the position of the variant sequence is confirmed by comparing each value of the codes obtained by designating C, T, A, and G of the nucleotide sequence of a specific DNA fragment as 00, 01, 10, and 11, respectively.


<110>     SON, In sik

<120>     A METHOD CODING STANDARDIZATION OF DNA AND A BIOTECHNOLOGICAL USE
          OF THE METHOD

<130>     OP19-0006HSPCT

<150>     KR 10-2019-0025377
<151>     2019-03-05

<160>     7

<170>     KopatentIn 2.0

<210>     1
<211>     32
<212>     DNA
<213>     Artificial Sequence

<220>
<223>     Oligonucleotide


<400>     1
agagctcgcg ccggagttct caatgcaaga gc                                     32


<210>     2
<211>     32
<212>     DNA
<213>     Artificial Sequence

<220>
<223>     Oligonucleotide


<400>     2
gcggcggtgg cctgaagtct ggcggtggcc cc                                     32


<210>     3
<211>     32
<212>     DNA
<213>     Artificial Sequence

<220>
<223>     Oligonucleotide


<400>     3
gcggcggtgg ccagaagtct cgcggtggcg gc                                     32


<210>     4
<211>     32
<212>     DNA
<213>     Artificial Sequence

<220>
<223>     Oligonucleotide


<400>     4

gtggaggcgg tggccagtct cgcggtggcg gc                                    32

<210>    5
<211>    32
<212>    DNA
<213>    Artificial Sequence

<220>
<223>    Oligonucleotide

<400>    5
gtggcggtgg ccagcatagt ggcggtggcc ag                                    32

<210>    6
<211>    32
<212>    DNA
<213>    Artificial Sequence

<220>
<223>    Oligonucleotide

<400>    6
gtggaggcgg tggccgtgga ggcggaggcc gc                                    32

<210>    7
<211>    9
<212>    DNA
<213>    Artificial Sequence

<220>
<223>    Oligonucleotide

<400>    7
gcggtggcg                                                               9